# EUROPEAN PATENT SPECIFICATION

(11) **EP 4 168 092 B1**
(45) Date of publication and mention of the grant of the patent: **27.05.2026**
(21) Application number: 20737810.0
(22) Date of filing: 22.06.2020
(51) Int. Cl.: A61M 25/10, A61M 25/00

(54) **BALLOON CATHETER WITH PRECISION DRUG-ELUTING CAPABILITY**
BALLONKATHETER MIT PRÄZISIONS-ARZNEIMITTELFREISETZENDER FÄHIGKEIT
CATHÉTER À BALLONNET AVEC CAPACITÉ D'ÉLUTION DE MÉDICAMENT DE PRÉCISION

(43) Date of publication of application: 26.04.2023
(73) Proprietor: C. R. Bard, Inc., Franklin Lakes, NJ 07417 (US)
(72) Inventor: SIMMONS, Brandon, Tempe, AZ 85282 (US)
(74) Representative: Hoffmann Eitle
(86) International application number: PCT/US2020/038911
(87) International publication number: WO 2021/262134

(56) References cited:
- WO-A1-2008/109114
- WO-A1-96/40346
- WO-A1-97/42998
- US-A1- 2007 250 035

## Description

### BACKGROUND

Balloon dilatation catheters are medical devices used to treat lesions in vessels. In the course of such treatment, it is sometimes desirable to deliver a substance, such as a drug, to the lesion undergoing treatment to improve the outcome. Past proposals for so-called "drug-eluting" balloon catheters have been made, but all suffer from considerable complexity, which makes such balloon catheters costly to manufacture and difficult to use. This includes an inability to target delivery of the drug to specific locations in a precise manner to ensure that a desired treatment outcome is achieved.

WO 2008/109114 A1 discloses a medical device for locally administering therapeutic agents. The device includes a balloon catheter with conduits external to the balloon and ports in the conduit wherein the conduits provide adequate sealing and sufficient penetration of the body vessel wall.

### SUMMARY OF THE INVENTION

The present invention is directed to the medical device of claim 1. The dependent claims refer to preferred embodiments.

### SUMMARY OF THE DISCLOSURE

An object of the disclosure is a balloon catheter with eluting capability provided by one or more tubes including one or more delivery ports for delivering a therapeutic agent, extending alongside an outer surface of the balloon, with the tube(s) being in communication with a lumen in an associated multi-lumen shaft.

The disclosure relates to a balloon catheter including a shaft having a proximal end portion and a distal end portion. The shaft includes a plurality of lumens, and an inflatable balloon mounted on the distal end portion of the shaft. A tube is associated with each of the plurality of lumens, and extends alongside the inflatable balloon. The tubes include one or more delivery ports for delivering a therapeutic agent to an adjacent vascular lesion.

In one embodiment, the plurality of lumens surround a central lumen of the shaft. Each tube may have a diameter that is less than a diameter of the central lumen. The plurality of lumens may comprise eight lumens.

Each tube may include one or more delivery ports for releasing a fluid delivered to the tube via an associated one of the plurality of lumens. The delivery ports may correspond to a cylindrical barrel portion of the inflatable balloon.

Each tube may include a closed end adjacent a distal end of the shaft. Each tube may also be bonded to an outer surface of the inflatable balloon. Adjacent tubes are spaced apart in a circumferential direction along an outer surface of the inflatable balloon. Adjacent tubes may also be closer to each other in a deflated condition of the inflatable balloon than in an inflated condition of the inflatable balloon. The shaft may also include a single proximal lumen in fluid communication with an open proximal end of each of the plurality of lumens.

In accordance with a further aspect of the disclosure, a balloon catheter is provided. The balloon catheter includes a shaft having a proximal end portion and a distal end portion. The shaft includes at least one lumen. An inflatable balloon is mounted on the distal end portion of the shaft. At least one tube extends along and is attached to an outer surface of the inflatable balloon. The at least one tube is in fluid communication with the at least one lumen and has a closed distal end.

In one embodiment, the shaft comprises a plurality of lumens, and a plurality of tubes extend alongside the balloon. Each of the plurality of tubes is in fluid communication with one of the plurality of lumens and has a closed distal end. The plurality of lumens may surround a central lumen of the shaft. The at least one tube may have a diameter that is less than a diameter of the central lumen, and the plurality of lumens may comprise eight lumens. The shaft may also include a single proximal lumen in fluid communication with an open proximal end of each of the plurality of lumens.

Each tube may include one or more delivery ports for releasing a fluid delivered to the tube via an associated one of the plurality of lumens. The delivery ports may correspond to a cylindrical barrel portion of the inflatable balloon. Each tube is bonded to an outer surface of the inflatable balloon. A plurality of tubes may be spaced apart in a circumferential direction along an outer surface of the inflatable balloon.

This disclosure also pertains to a balloon catheter including an inflatable balloon with a proximal tapered end portion, a distal tapered end portion, and an intermediate barrel portion therebetween. A plurality of tubes extend along an outer surface of the inflatable balloon along the at least a proximal tapered end portion of the inflatable balloon and the central barrel portion of the inflatable balloon. A portion of each tube extends along the intermediate barrel portion of the inflatable balloon including one or more delivery ports.

The plurality of tubes may be spaced apart in a circumferential direction along an outer surface of the inflatable balloon. Adjacent ones of the plurality of tubes may be closer to each other in a deflated condition of the inflatable balloon than in an inflated condition of the inflatable balloon. The plurality of tubes may be bonded to an outer surface of the inflatable balloon. The shaft may also include a single proximal lumen in fluid communication with an open proximal end of each of the plurality of lumens.

### BRIEF DESCRIPTION OF THE DRAWINGS

The above and further advantages of the disclosure may be better understood by referring to the following description in conjunction with the accompanying drawings in which:
Figure 1 is a side view of an inflatable balloon for use in connection with a balloon catheter.
Figure 1A is an enlarged, cutaway view of the inflatable balloon of Figure 1.
Figure 1B is a partial cross-sectional view of the inflatable balloon of Figure 1, taken along line 1B-1B of Figure 1A.
Figure 2 is a side view of a balloon catheter.
Figures 2A, 2B, and 2C are cross-sectional views taken along lines 2A-2A, 2B-2B, and 2C-2C of Figure 2, respectively.
Figure 2D is a cross-sectional view taken along line 2D-2D of Figure 2.
Figure 2E is a cross-sectional view of the balloon of Figure 2D in an uninflated, folded condition.
Figure 3 is a partially cutaway, partially cross-sectional view of the catheter of Figure 2.
Figure 4 is a schematic view illustrating one manner of using the disclosed balloon catheter.
Figures 5, 5A, and 5B illustrate an alternate embodiment.

The dimensions of some of the elements may be exaggerated relative to other elements for clarity or several physical components may be included in one functional block or element. Further, sometimes reference numerals may be repeated among the drawings to indicate corresponding or analogous elements. Moreover, some of the items depicted in the drawings may be combined into a single function.

### DETAILED DESCRIPTION

In the following detailed description, numerous specific details are set forth to provide a thorough understanding of the present invention. The disclosed embodiments may be practiced without these specific details. In other instances, well-known methods, procedures, components, or structures may not have been described in detail so as not to obscure the present invention.

Certain features of the invention that are, for clarity, described in the context of separate embodiments, may also be provided in combination in a single embodiment. Conversely, various features of the invention that are, for brevity, described in the context of a single embodiment, may also be provided separately or in any suitable sub-combination.

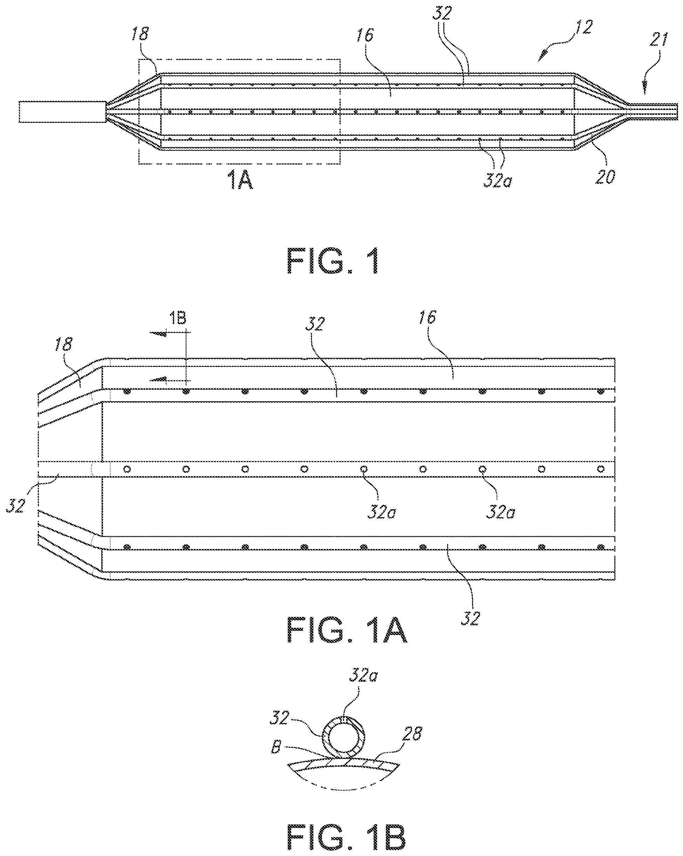

With reference to Figures 1, 1A, 2, 2A, 2B, 2C, and 3, provided is a catheter 10 having a shaft 14 with a distal end portion 11 having an inflatable balloon 12 mounted thereon. The balloon 12 has an intermediate portion 16, or "barrel," and end portions 18, 20. In one embodiment, the end portions 18, 20 reduce in diameter to join the intermediate portion 16 to the catheter shaft 14 (and thus portions 18, 20 are generally termed "cones" or "cone portions"). The balloon 12 is sealed at balloon ends (proximal end 15a and distal end 15b) to allow the inflation of the balloon 12 via one or more inflation lumens 17 extending within catheter shaft 14 and communicating with the interior of the balloon 12.

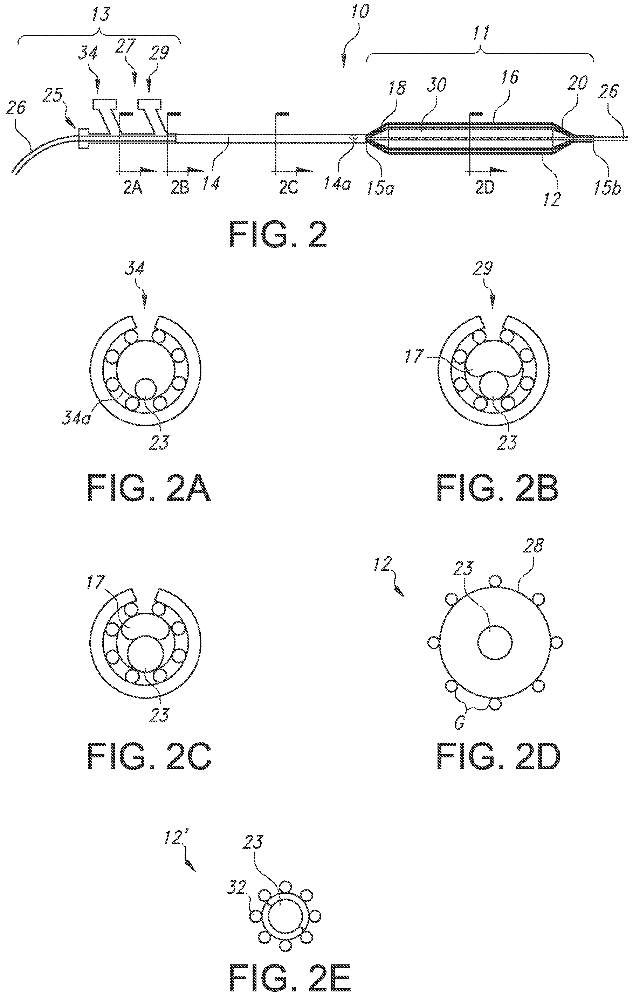

The catheter 10 also includes a guidewire lumen 23 formed by a shaft 24, which may be within the shaft 14 and, more particularly, within the inflation lumen 17. This lumen 23 directs the guidewire 26 through the catheter 10, and along the distal end portion of which the balloon 12 may be located, including through a tip 21 distal of the distal end 15b of the balloon 12. As illustrated in Figure 2, this guidewire 26 may extend through the proximal end portion of the catheter 10 via a first port 25 of a connector or hub 27 at a proximal end portion 13 of the shaft 14 into the lumen 23 to achieve an "over the wire" (OTW) arrangement, but could also be provided in a "rapid exchange" (RX) configuration (in which the guidewire 26 exits from the shaft 14 at an optional lateral opening (see phantom line indication 14a in Figure 2) closer to the distal end but proximal of balloon 12) or else is fed through the tip at a passage distally of the balloon 12 ("short" RX; not shown). A second port 29 may also be associated with catheter 10, such as by way of connector 27, for introducing a fluid (e.g., saline, a contrast agent, or both) into the interior compartment of the balloon 12 via the inflation lumen 17.

Inflatable balloon 12 may include a single or multi-layered balloon wall 28 (see Figures 1B and 2D) forming the interior for receiving the inflation fluid. The balloon 12 may be a non-compliant balloon having a balloon wall that maintains its size and shape in one or more directions when the balloon is inflated. The balloon 12 in such case also has a pre-determined surface area that remains constant during and after inflation, also has a pre-determined length and pre-determined diameter that each, or together, remain constant during and after inflation. However, the balloon 12 could be semi-compliant or compliant instead, depending on the particular use. The balloon 12 can have any of a variety of diameters ranging from 1.25-40 mm or 2.0-8.0 mm. In any of the embodiments set out herein, inflatable balloon 12 can have any of a variety of lengths such as 10-300 mm or 20-300 mm. Longer balloons may be particularly useful for treating peripheral lesions, which often have long diseased portions.

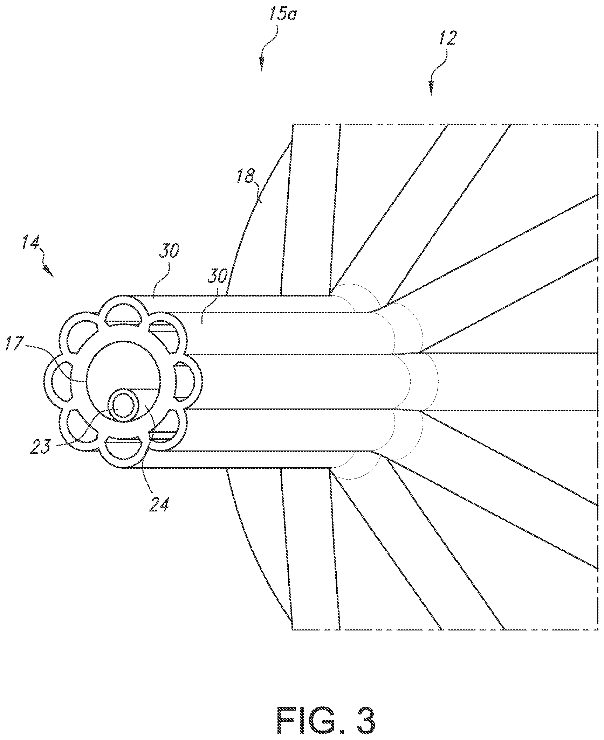

According to a first aspect of the disclosure, and with reference to Figures 1, 1A, and 2A-2D, and 3, the shaft 14 may include a plurality of lumens 30, each in fluid communication with a tube 32 extending alongside the inflatable balloon 12. Eight such tubes 32 and a corresponding number of lumens 30 are shown in the illustrated embodiment, but the number could be smaller (including possibly only one) or greater. The lumens 30 of the shaft 14 may surround the central inflation lumen, as shown in Figure 3, and may be arranged to provide the shaft with a petaloidal cross-section, which is optional. A proximal end of each lumen 30 is in fluid communication with a port 34 adjacent the hub 27, which may communicate with an annulus 34a associated with each lumen, and a distal end of each lumen is closed adjacent to a distal end 15b of the balloon 12, such as at the tip 21 or proximal thereof. As further understood from Figure 3, each lumen 30 is of a smaller diameter and cross-sectional area than the inflation lumen 17 in the illustrated embodiment.

In the region where at which the lumens 30 reach the proximal end 15a of the balloon 12, the tubes 32 are connected thereto. The tubes 32 extend initially along the proximal end portion 18 of the balloon 12, along the intermediate portion 16 of the balloon, and optionally continue by extending along the distal end portion 20 of the balloon. The tubes 32 may comprise discrete, hollow cylinders, which are bonded to an external or outer surface of the balloon wall 28 (note reference character B in Figure 1B). The bonding may be achieved using an adhesive and/or heat and pressure (such as may be achieved using a die or press).

As can be understood from Figures 2D and 2E, the tubes 32 are spaced apart with gaps G in between adjacent ones. As can be appreciated, the tubes 32 may become spaced further apart when the balloon 12 is in an inflated condition, and move closer together and even possibly touch peripherally when the balloon 12 is in a uninflated or folded condition (see reference number 12' in Figure 2E). The tubes 32 may have a diameter and cross-sectional area generally corresponding to that of the associated lumens 30 of the shaft 14.

As perhaps best understood from Figures 1A and 1B, the portion of each tube 32 along the intermediate "barrel" portion 16, of the balloon 12 is provided with one or more delivery ports 32a. The delivery port(s) 32a may be spaced apart in a longitudinal direction (such as equidistantly), and are formed on the radially outermost portion of the tube 32 to ensure close proximity to an adjacent lesion during inflation of the balloon 12 in a body vessel, as shown in Figure 1B. In other words, the delivery port(s) 32a are generally located opposite the location of the bond B formed between the radially innermost portion of tube 30 and the outer surface of the balloon wall 28, and arranged such that a centerline is generally perpendicular with a longitudinal axis of the tube 32.

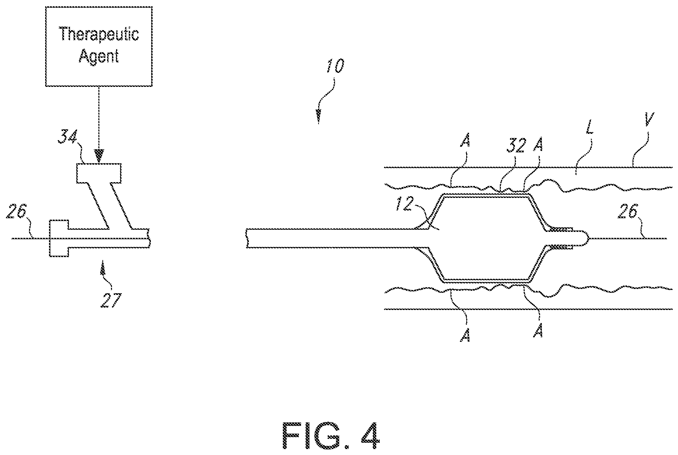

Turning to Figure 4, in use, the physician may maneuver the catheter 10 along the guidewire 26 until inflatable balloon 12 reaches the desired position near the site of a lesion L in a vessel V. Typically, inflation of the balloon 12 locates the tubes 32 in close proximity to the lesion L. Provided a sufficiently rigid material is used, the tubes 32 may even be used to firmly press into or crack the lesion L. In the case of a highly or "super" compliant balloon, portions of the balloon 12 in the gap G (Figure 2D) between the tubes 32 may actually expand or bulge beyond the radial extent of the tubes, and actually then move into contact with the lesion L for stability (which adjacent bulges may also form boundaries in a circumferential direction and create a channel including the tube for controlled delivery of a selected therapeutic agent to a specific portion of the lesion).

Regardless of whether scoring functionality or a super compliant balloon is provided, which are both entirely optional, once the balloon 12 is inflated, a drug or like therapeutic agent A may be delivered from a source, via port 34. The agent once delivered to the catheter 10 may travel via lumens 30 to the tubes 32, and elute from delivery ports 32a in one or more radial directions and one or more locations (depending on the selected arrangement) to provide a desired treatment effect to the adjacent lesion L.

As can be appreciated, strategic spacing or positioning of the one or more tubes 32 and associated delivery port(s) 32a may provide a precise manner of control of delivery of the drug or therapeutic agent, especially for a typical asymmetrical or irregular lesion L (as shown in Figure 4). For instance, the one or more tube(s) 32 may be provided in a desired number and location to provide a treatment direction or vector (such as for instance, approximately 180 degrees apart in the case of two tubes, approximately 120 degrees apart in the case of three, approximately 90 degrees apart in the case of four, approximately 60 degrees apart in the case of six tubes, approximately 45 degrees apart in the case of eight tubes, and so on and so forth). Furthermore, the delivery ports 32a may be spaced in the longitudinal direction and provided in such number and in the same or different diameters or sizes to regulate and control the delivery of an eluted substance. As a result, a far more precise treatment may be provided during an interventional procedure using the catheter 10, and a potentially better outcome achieved.

Once lesion treatment with catheter 10 is complete, the physician deflates inflatable balloon 12. This allows tubes 32 to relax away from the lesion L and from the wall of the vessel V, and essentially assume the condition similar to that shown in Figure 2E. The catheter 10 may then be removed or repositioned as desired to effect further treatment, as necessary or desired.

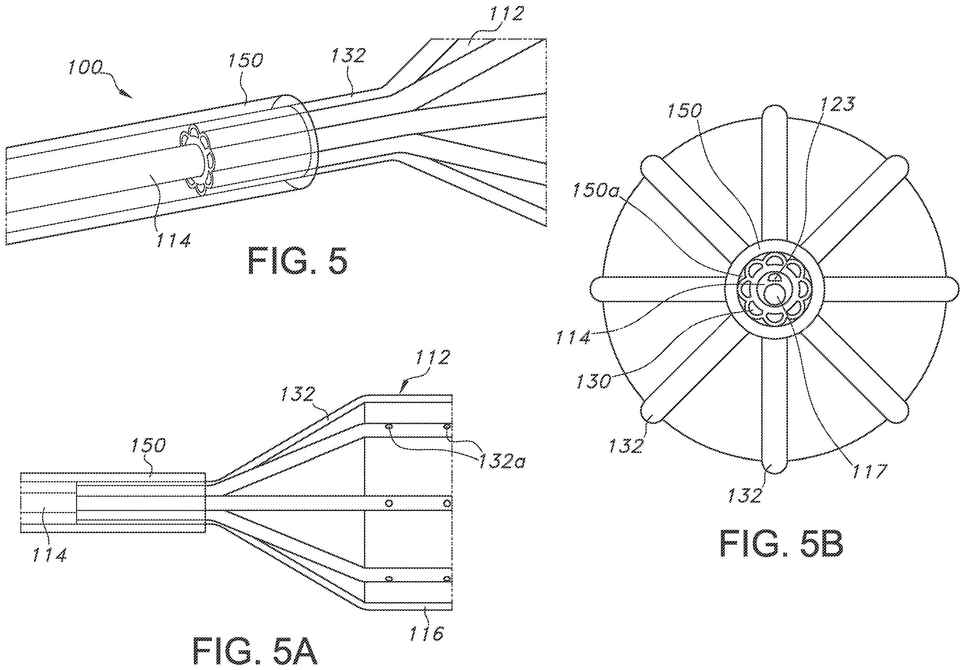

An alternate embodiment is shown in Figures 5, 5A, and 5B. In this embodiment, a catheter 100 with a similar balloon 112 as described above is provided. In this version, the tubes 132 forming the lumens 130 for delivering the treatment agent extend proximally of the balloon 112, and are at least partially covered by an outer tubular shaft 150, which may be bonded in place and extend to the proximal end of the catheter 100. The tubes 132 are bonded to the external surface of the balloon 112 and include delivery ports 132, including along the barrel portion 116 of the balloon 112.

This shaft 150 thus provides a single annular lumen 150a in communication with the lumens 130. The single annular lumen 150a may at a proximal end communicate with the port associated with the proximal hub (not shown) for receiving a therapeutic agent. An inner shaft 114 coaxial with the outer shaft 150 may include additional lumens, such as an inflation lumen 117 and/or guidewire lumen 123.

Suitable drugs or therapeutic agents may include antimicrobial agents such as, for example, from triclosan from triclosan, chlorhexidine, nitrofurazone, benzalkonium chlorides, silver salts and antibiotics such as rifampin, gentamycin and minocyclin and combinations thereof, among others. In certain embodiments, antimicrobial agents may include triclosan, chlorhexidine and salts or combinations thereof. Anti-inflammatory agents include steroidal and non-steroidal anti-inflammatory agents. Examples of nonsteroidal anti-inflammatory drugs include aminoarylcarboxylic acid derivatives such as enfenamic acid, etofenamate, flufenamic acid, isonixin, meclofenamic acid, mefanamic acid, niflumic acid, talniflumate, terofenamate and tolfenamic acid; arylacetic acid derivatives such as acemetacin, alclofenac, amfenac, bufexamac, cinmetacin, clopirac, diclofenac sodium, etodolac, felbinac, fenclofenac, fenclorac, fenclozic acid, fentiazac, glucametacin, ibufenac, indomethacin, isofezolac, isoxepac, lonazolac, metiazinic acid, oxametacine, proglumetacin, sulindac, tiaramide, tolmetin and zomepirac; arylbutyric acid derivatives such as bumadizon, butibufen, fenbufen and xenbucin; arylcarboxylic acids such as clidanac, ketorolac and tinoridine; arylpropionic acid derivatives such as alminoprofen, benoxaprofen, bucloxic acid, carprofen, fenoprofen, flunoxaprofen, flurbiprofen, ibuprofen, ibuproxam, indoprofen, ketoprofen, loxoprofen, miroprofen, naproxen, oxaprozin, piketoprofen, pirprofen, pranoprofen, protizinic acid, suprofen and tiaprofenic acid; pyrazoles such as difenamizole and epirizole; pyrazolones such as apazone, benzpiperylon, feprazone, mofebutazone, morazone, oxyphenbutazone, phenybutazone, pipebuzone, propyphenazone, ramifenazone, suxibuzone and thiazolinobutazone; salicylic acid and its derivatives such as acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamine a-acetic acid, salicylsulfuric acid, salsalate and sulfasalazine; thiazinecarboxamides such as droxicam, isoxicam, piroxicam and tenoxicam; others such as E-acetamidocaproic acid, s-adenosylmethionine, 3-amino-4- hydroxybutyric acid, amixetrine, bendazac, benzydamine, bucolome, difenpiramide, ditazol, emorfazone, guaiazulene, nabumetone, nimesulide, orgotein, oxaceprol, paranyline, perisoxal, pifoxime, proquazone, proxazole and tenidap; and pharmaceutically acceptable salts thereof.

Examples of steroidal anti-inflammatory agents (glucocorticoids) include 21-acetoxyprefnenolone, alclometasone, algestone, amicinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumehtasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol priopionate, halometasone, halopredone acetate, hydrocortamate, hydrocortisone, loteprednol etabonate, mazipredone, medrysone, meprednisone, methyolprednisolone, mometasone furoate, paramethasone, prednicarbate, prednisolone, prednisolone 25-diethylaminoacetate, prednisone sodium phosphate, prednisone, prednival, prednylidene, rimexolone, tixocortal, triamcinolone, triamcinolone acetonide, triamcinolone benetonide, triamcinolone hexacetonide, and pharmaceutically acceptable salts thereof.

Analgesic agents include narcotic and non-narcotic analgesics. Narcotic analgesic agents include alfentanil, allylprodine, alphaprodine, anileridine, benzylmorphine, bezitramide, buprenorphine, butorphanol, clonitazene, codeine, codeine methyl bromide, codeine phosphate, codeine sulfate, desomorphine, dextromoramide, dezocine, diampromide, dihydrocodeine, dihydrocodeinone enol acetate, dihydromorphine, dimenoxadol, dimepheptanol, dimethylthiambutene, dioxaphetyl butyrate, dipipanone, eptazocine, ethoheptazine, ethylmethlythiambutene, ethylmorphine, etonitazene, fentanyl, hydrocodone, hydromorphone, hydroxypethidine, isomethadone, ketobemidone, levorphanol, lofentanil, meperidine, meptazinol, metazocine, methadone hydrochloride, metopon, morphine, myrophine, nalbuphine, narceine, nicomorphine, norlevorphanol, normethadone, normorphine, norpipanone, opium, oxycodone, oxymorphone, papaveretum, pentazocine, phenadoxone, phenazocine, pheoperidine, piminodine, piritramide, proheptazine, promedol, properidine, propiram, propoxyphene, rumifentanil, sufentanil, tilidine, and pharmaceutically acceptable salts thereof. Non-narcotic analgesics include aceclofenac, acetaminophen, acetaminosalol, acetanilide, acetylsalicylsalicylic acid, alclofenac, alminoprofen, aloxiprin, aluminum bis(acetylsalicylate), aminochlorthenoxazin,2-amino-4-picoline, aminopropylon, aminopyrine, ammonium salicylate, amtolmetin guacil, antipyrine, antipyrine salicylate, antrafenine, apazone, aspirin, benorylate, benoxaprofen, benzpiperylon, benzydamine, bermoprofen, brofenac, p-bromoacetanilide, 5-bromosalicylic acid acetate, bucetin, bufexamac, bumadizon, butacetin, calcium acetylsalicylate, carbamazepine, carbiphene, carsalam, chloralantipyrine, chlorthenoxazin( e ), choline salicylate, cinchophen, ciramadol, clometacin, cropropamide, crotethamide, dexoxadrol, difenamizole, diflunisal, dihydroxyaluminum acetylsalicylate, dipyrocetyl, dipyrone, emorfazone, enfenamic acid, epirizole, etersalate, ethenzamide, ethoxazene, etodolac, felbinac, fenoprofen, floctafenine, flufenamic acid, fluoresone, flupirtine, fluproquazone, flurbiprofen, fosfosal, gentisic acid, glafenine, ibufenac, imidazole salicylate, indomethacin, indoprofen, isofezolac, isoladol, isonixin, ketoprofen, ketorolac, p-lactophenetide, lefetamine, loxoprofen, lysine acetylsalicylate, magnesium acetylsalicylate, methotrimeprazine, metofoline, miroprofen, morazone, morpholine salicylate, naproxen, nefopam, nifenazone, 5' nitro-2' propoxyacetanilide, parsalmide, perisoxal, phenacetin, phenazopyridine hydrochloride, phenocoll, phenopyrazone, phenyl acetylsalicylate, phenyl salicylate, phenyramidol, pipebuzone, piperylone, prodilidine, propacetamol, propyphenazone, proxazole, quinine salicylate, ramifenazone, rimazolium metilsulfate, salacetamide, salicin, salicylamide, salicylamide a-acetic acid, salicylsulfuric acid, salsalte, salverine, simetride, sodium salicylate, sulfamipyrine, suprofen, talniflumate, tenoxicam, terofenamate, tetradrine, tinoridine, tolfenamic acid, tolpronine, tramadol, viminol, xenbucin, zomepirac, and pharmaceutically acceptable salts thereof.

Local anesthetic agents include amucaine, amolanone, amylocaine hydrochloride, benoxinate, benzocaine, betoxycaine, biphenamine, bupivacaine, butacaine, butaben, butanilicaine, butethamine, butoxycaine, carticaine, chloroprocaine hydrochloride, cocaethylene, cocaine, cyclomethycaine, dibucaine hydrochloride, dimethisoquin, dimethocaine, diperadon hydrochloride, dyclonine, ecgonidine, ecgonine, ethyl chloride, beta-eucaine, euprocin, fenalcomine, fomocaine, hexylcaine hydrochloride, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine hydrochloride, phenol, piperocaine, piridocaine, polidocanol, pramoxine, prilocaine, procaine, propanocaine, proparacaine, propipocaine, propoxycaine hydrochloride, pseudococaine, pyrrocaine, ropavacaine, salicyl alcohol, tetracaine hydrochloride, tolycaine, trimecaine, zolamine, and pharmaceutically acceptable salts thereof.

Antispasmodic agents include alibendol, ambucetamide, aminopromazine, apoatropine, bevonium methyl sulfate, bietamiverine, butaverine, butropium bromide, n-butylscopolammonium bromide, caroverine, cimetropium bromide, cinnamedrine, clebopride, coniine hydrobromide, coniine hydrochloride, cyclonium iodide, difemerine, diisopromine, dioxaphetyl butyrate, diponium bromide, drofenine, emepronium bromide, ethaverine, feclemine, fenalamide, fenoverine, fenpiprane, fenpiverinium bromide, fentonium bromide, flavoxate, flopropione, gluconic acid, guaiactamine, hydramitrazine, hymecromone, leiopyrrole, mebeverine, moxaverine, nafiverine, octamylamine, octaverine, oxybutynin chloride, pentapiperide, phenamacide hydrochloride, phloroglucinol, pinaverium bromide, piperilate, pipoxolan hydrochloride, pramiverin, prifinium bromide, properidine, propivane, propyromazine, prozapine, racefemine, rociverine, spasmolytol, stilonium iodide, sultroponium, tiemonium iodide, tiquizium bromide, tiropramide, trepibutone, tricromyl, trifolium, trimebutine, n,n- 1 trimethyl-3,3-diphenyl-propylamine, tropenzile, trospium chloride, xenytropium bromide, and pharmaceutically acceptable salts thereof.

In certain embodiments, therapeutic agents for reducing pain or discomfort may be selected from ketorolac and pharmaceutically acceptable salts thereof (e.g., the tromethamine salt thereof, sold under the commercial name Torado^{®}), 4-diethylamino-2-butynylphenylcyclohexylglycolate and pharmaceutically acceptable salts thereof (e.g., 4-diethylamino-2-butynylphenylcyclohexylglycolate hydrochloride, also known as oxybutynin chloride, sold under the commercial name Ditropang^{®}), and combinations thereof. The amount of therapeutic agent present, will depend, for example, upon the efficacy of the therapeutic agent employed, the release rate, and so forth. One skilled in the art can readily determine an appropriate therapeutic agent loading to achieve the desired outcome.

## Claims

1. A medical device, comprising:
a shaft (14) having a proximal end portion and a distal end portion, the shaft including a plurality of lumens (30);
an inflatable balloon (12) mounted on the distal end portion of the shaft (14); and
a tube (32) associated with each of the plurality of lumens (30), the tube (32) extending alongside the inflatable balloon (12) and having one or more delivery ports (34), wherein each tube (32) is bonded to an outer surface of the inflatable balloon (12).

2. The medical device of claim 1, wherein the plurality of lumens (30) surround a central lumen of the shaft (14).

3. The medical device of claim 2, wherein each tube (32) has a diameter that is less than a diameter of the central lumen.

4. The medical device of claim 1, wherein the plurality of lumens (30) comprise eight lumens.

5. The medical device of claim 1, wherein each tube (32) includes one or more delivery ports (132) for releasing a fluid delivered to the tube via an associated one of the plurality of lumens.

6. The medical device of claim 5, wherein the delivery ports correspond to a cylindrical barrel portion of the inflatable balloon.

7. The medical device of claim 1, wherein each tube (32) includes a closed end adjacent a distal end of the shaft (14).

8. The medical device of claim 1, wherein adjacent tubes (32) are spaced apart in a circumferential direction along an outer surface of the inflatable balloon (12).

9. The medical device of claim 8, wherein the plurality of tubes (32) are closer to each other in a deflated condition of the inflatable balloon (12) than in an inflated condition of the inflatable balloon (12).

10. The medical device of any of claims 1-9, wherein the proximal end portion (13) of the shaft (14) includes a single lumen (23) in communication with the plurality of lumens, which are located at the distal end portion of the shaft.

## Patentansprüche

1. Medizinische Vorrichtung, umfassend:
einen Schaft (14) mit einem proximalen Endabschnitt und einem distalen Endabschnitt, wobei der Schaft eine Vielzahl von Lumen (30) aufweist;
einen aufblasbaren Ballon (12), der am distalen Endabschnitt des Schafts (14) angebracht ist; und
einen Schlauch (32), die jedem der Vielzahl von Lumen (30) zugeordnet ist, wobei sich der Schlauch (32) entlang des aufblasbaren Ballons (12) erstreckt und eine oder mehrere Zuführungsöffnungen (34) aufweist, wobei jeder Schlauch (32) mit einer Außenfläche des aufblasbaren Ballons (12) verbunden ist.

2. Medizinische Vorrichtung nach Anspruch 1, wobei die Vielzahl von Lumen (30) ein zentrales Lumen des Schafts (14) umgeben.

3. Medizinische Vorrichtung nach Anspruch 2, wobei jeder Schlauch (32) einen Durchmesser aufweist, der kleiner ist als der Durchmesser des zentralen Lumens.

4. Medizinische Vorrichtung nach Anspruch 1, wobei die Vielzahl von Lumen (30) acht Lumen umfassen.

5. Medizinische Vorrichtung nach Anspruch 1, wobei jeder Schlauch (32) eine oder mehrere Abgabemündungen (132) aufweist, um ein Fluid abzugeben, das der Schlauch über eines der zugehörigen Lumen der Vielzahl von Lumen zugeführt wird.

6. Medizinische Vorrichtung nach Anspruch 5, wobei die Zufuhröffnungen einem zylindrischen Zylinderabschnitt des aufblasbaren Ballons entsprechen.

7. Medizinische Vorrichtung nach Anspruch 1, wobei jeder Schlauch (32) ein geschlossenes Ende aufweist, das an das distale Ende des Schafts (14) angrenzt.

8. Medizinische Vorrichtung nach Anspruch 1, wobei benachbarte Schläuche (32) in Umfangsrichtung entlang einer Außenfläche des aufblasbaren Ballons (12) voneinander beabstandet sind.

9. Medizinische Vorrichtung nach Anspruch 8, wobei die Vielzahl von Schläuchen (32) im entleerten Zustand des aufblasbaren Ballons (12) näher beieinander liegen als im aufgeblasenen Zustand des aufblasbaren Ballons (12).

10. Medizinische Vorrichtung nach einem der Ansprüche 1-9, wobei der proximale Endabschnitt (13) des Schafts (14) ein einzelnes Lumen (23) aufweist, das mit der Vielzahl von Lumen in Verbindung steht, die sich am distalen Endabschnitt des Schafts befinden.

## Revendications

1. Dispositif médical, comprenant :
une tige (14) présentant une partie d'extrémité proximale et une partie d'extrémité distale, la tige incluant une pluralité de lumières (30) ;
un ballonnet gonflable (12) monté sur la partie d'extrémité distale de la tige (14) ; et
un tube (32) associé à chacune des lumières de la pluralité de lumières (30), le tube (32) s'étendant le long du ballonnet gonflable (12) et présentant un ou plusieurs orifices de distribution (34), dans lequel chaque tube (32) est fixé à une surface externe du ballonnet gonflable (12).

2. Dispositif médical selon la revendication 1, dans lequel la pluralité de lumières (30) entoure une lumière centrale de la tige (14).

3. Dispositif médical selon la revendication 2, dans lequel chaque tube (32) présente un diamètre qui est inférieur à un diamètre de la lumière centrale.

4. Dispositif médical selon la revendication 1, dans lequel la pluralité de lumières (30) comprend huit lumières.

5. Dispositif médical selon la revendication 1, dans lequel chaque tube (32) inclut un ou plusieurs orifices de distribution (132) pour libérer un fluide délivré au tube via une lumière associée de la pluralité de lumières.

6. Dispositif médical selon la revendication 5, dans lequel les orifices de distribution correspondent à une partie de barillet cylindrique du ballonnet gonflable.

7. Dispositif médical selon la revendication 1, dans lequel chaque tube (32) inclut une extrémité fermée adjacente à une extrémité distale de la tige (14).

8. Dispositif médical selon la revendication 1, dans lequel des tubes adjacents (32) se situent espacés les uns des autres dans une direction circonférentielle le long d'une surface externe du ballonnet gonflable (12).

9. Dispositif médical selon la revendication 8, dans lequel la pluralité de tubes (32) se situent plus proches les uns des autres dans un état dégonflé du ballonnet gonflable (12) que dans un état gonflé du ballonnet gonflable (12).

10. Dispositif médical selon l'une quelconque des revendications 1 à 9, dans lequel la partie d'extrémité proximale (13) de la tige (14) inclut une lumière unique (23) en communication avec la pluralité de lumières, lesquelles se situent au niveau de la partie d'extrémité distale de la tige.
